# EUROPEAN PATENT APPLICATION

(11) **EP 4 498 006 A1**
(43) Date of publication of application: **29.01.2025**
(21) Application number: 23775135.9
(22) Date of filing: 17.01.2023
(51) Int. Cl.: F24F 1/0076, F24F 1/0025, F24F 13/20, F24F 8/22, A61L 2/10, A61L 9/20

(54) **AIR CONDITIONER**

(30) Priority: 25.03.2022 KR 20220037516
(71) Applicant: LG Electronics Inc., Yeongdeungpo-gu Seoul 07336 (KR)
(72) Inventor: KIM, Junghyeon, Seoul 08592 (KR); SONG, Seungyong, Seoul 08592 (KR); JEONG, Suhyeon, Seoul 08592 (KR); PARK, Geunyoung, Seoul 08592 (KR); SHIN, Sooyeon, Seoul 08592 (KR)
(74) Representative: Ter Meer Steinmeister & Partner
(86) International application number: PCT/KR2023/000818
(87) International publication number: WO 2023/182632

(57) **Abstract**

An air conditioner according to the present disclosure includes: a case that defines an inlet and an outlet; a cross-flow fan that is disposed in the case and causes a flow of air; a heat exchanger that is disposed in the case and configured to exchange heat with flowing air; and a sterilization module that is disposed in the case in a direction of a rotation axis of the cross-flow fan, the sterilization module being configured to irradiate ultraviolet light to the cross-flow fan, wherein the sterilization module includes a plurality of ultraviolet lamps arranged to be spaced apart in the direction of the rotation axis of the cross-flow fan. Accordingly, the sterilization module including the plurality of ultraviolet lamps may be disposed to cover an entire length of the cross-flow fan in the direction of the rotation axis so as to allow an entire area of the cross-flow fan to be thoroughly sterilized, thereby providing the air conditioner including the sterilization module capable of covering the cross-flow fan.

## Description

### [Technical Field]

The present disclosure relates to an air conditioner, and more particularly, to an air conditioner including a sterilization module that can uniformly irradiate ultraviolet light to a cross-flow fan.

### [Background Art]

An air conditioner is a machine that controls and circulates air for heating, ventilation, and/or cooling. Such an air conditioner generally includes a fan, a heat exchanger, a chamber, a damper, etc.

Fans used in air conditioners can be classified into various types according to the type of air conditioner. Among them, a cross-flow fan (CFF), which is a fan having one or more fan blocks consisting of a plurality of blades curved in the direction of rotation, is configured to be elongated in an axial direction.

Meanwhile, ultraviolet light (or rays) is used to sterilize harmful bacteria such as E. coli, Staphylococcus aureus, and mold in an eco-friendly way. Ultraviolet light is widely used in various products, including air conditioners, to provide sterilization functions. In this case, when a human body is exposed to an excessive amount of ultraviolet light, it can cause skin cancer, cataracts, etc. Therefore, extra caution is required not only when using the product, but also when designing and manufacturing the product.

In the case of an air conditioner, water generated during heat exchange cannot easily evaporate due to the structure of the air conditioner, which is a major cause of bacterial growth. In particular, a fan having multiple narrow and deep crevices are more vulnerable to bacterial growth, and due to its structure, it is not suitable for cleaning and maintenance.

A related art air conditioner, as disclosed in Korean Patent Publication No. 10-2022-0011194A, includes a fan housing having a UV LED irradiation module. However, when a cross-flow fan is used, the related art air conditioner does not provide a sterilization module that can cover the entire area of the cross-flow fan.

### [Disclosure]

### [Technical Problem]

One aspect of the present disclosure is to provide an air conditioner including a sterilization module that can irradiate ultraviolet light to a cross-flow fan of the air conditioner without any missing part while covering an entire length of the cross-flow fan.

Another aspect of the present disclosure is to provide an air conditioner including a sterilization module with improved installation and access convenience.

Yet another aspect of the present disclosure is to provide an air conditioner including a sterilization module that is easily applicable to air conditioners that have been manufactured and/or distributed.

Yet another aspect of the present disclosure is to provide an air conditioner including a sterilization module that can be disposed adjacent to a fan that causes a flow of air without affecting the flow of air.

Yet another aspect of the present disclosure is to provide an air conditioner including a sterilization module that is easy to manufacture.

Yet another aspect of the present disclosure is to provide an air conditioner including a sterilization module that can expand an irradiation angle of ultraviolet light irradiated to a cross-flow fan in a limited inner space of the air conditioner while preventing the ultraviolet light, which can be harmful to a human body, from leaking to an outside of the air conditioner.

Yet another aspect of the present disclosure is to provide an air conditioner including a sterilization module that can uniformly irradiate ultraviolet light to a cross-flow fan having a long length in the direction of a rotation axis.

Yet another aspect of the present disclosure is to provide an air conditioner including a sterilization module with scalability to easily correspond to a cross-flow fan that varies in length depending on the capacity of the air conditioner.

The aspects of the present disclosure are not limited to the aspects described above, and other aspects not stated herein will be clearly understood by those skilled in the art from the following description.

### [Technical Solution]

To achieve the aforementioned aspects, an air conditioner according to an embodiment of the present disclosure includes: a case that defines an inlet and an outlet; a cross-flow fan that is disposed in the case and causes a flow of air; a heat exchanger that is disposed in the case and configured to exchange heat with flowing air; and a sterilization module that is disposed in the case in a direction of a rotation axis of the cross-flow fan, the sterilization module being configured to irradiate ultraviolet light to the cross-flow fan, wherein the sterilization module includes a plurality of ultraviolet lamps arranged to be spaced apart in the direction of the rotation axis of the cross-flow fan.

Thus, as the sterilization module of the air conditioner is disposed to cover the length in the direction of the rotation axis of the cross-flow fan, an entire area of the cross-flow fan may be thoroughly sterilized, thereby providing an air conditioner including a sterilization module capable of covering a cross-flow fan.

The case of the air conditioner according to the embodiment of the present disclosure may include: an upper case accommodated in a ceiling and in which the cross-flow fan is disposed; and a lower case disposed under the upper case so as to be exposed to an indoor space and in which the inlet and the outlet are disposed. The sterilization module may be disposed at a lower portion of the upper case. In addition, the lower case and the sterilization module may be detachably coupled to the upper case.

Thus, as the sterilization module is disposed at the lower portion of the upper case to facilitate a user's access upon removing the lower case exposed to the indoor space, an air conditioner with improved installation and access convenience may be provided.

The upper case of the air conditioner according to the embodiment of the present disclosure may include a guide housing that forms an air flow path from the cross-flow fan to the outlet. The guide housing may include a placement space formed as one surface opposite the cross-flow fan being bent. The sterilization module may further include an embedded portion embedded in the placement space to couple the sterilization module.

Thus, as the placement space of the sterilization module is produced through a simple bending or recess process regardless of the shape of typical air conditioner that has been manufactured and/or distributed, an air conditioner including a sterilization module that is easily applicable to air conditioners that have been manufactured and/or distributed may be provided.

In addition, although the sterilization module is disposed adjacent to the fan that causes a flow of air to sterilize the fan, the sterilization module is embedded so as not to interfere with the flow of air, an air conditioner including a sterilization module that is disposed in a manner that does not interfere with a flow of air may be provided.

The sterilization module of the air conditioner according to the embodiment of the present disclosure may further include: a PCB bar on which the plurality of ultraviolet lamps are disposed, the PCB bar being electrically connected to the plurality of ultraviolet lamps; a lamp case to which the PCB bar is coupled; and a module case to which the lamp case is coupled, the module case having a plurality of opening holes that correspond to the plurality of ultraviolet lamps.

Thus, as the lamp case is coupled to the module case after coupling the PCB bar to the lamp case, an air conditioner including a sterilization module that is easy to manufacture may be provided.

The module case of the air conditioner according to the embodiment of the present disclosure may include: a protruding portion protruding from a side of the inlet and the outlet in the plurality of opening holes so as to limit an irradiation angle of the side of the inlet and the outlet; and an inclined portion formed on an opposite side of the inlet and the outlet in the plurality of opening holes so as to expand an irradiation angle of the opposite side of the inlet and the outlet.

Thus, as the shape of an ultraviolet light outlet of the sterilization module is formed in consideration of the irradiation angle of ultraviolet light, an air conditioner including a sterilization module that can expand an irradiation angle of ultraviolet light irradiated to a cross-flow fan in a limited inner space of the air conditioner while preventing the ultraviolet light, which can be harmful to a human body, from leaking to an outside of the air conditioner may be provided.

A separation distance between adjacent ultraviolet lamps of the plurality of ultraviolet lamps of the air conditioner according to the embodiment of the present disclosure may have a predetermined ratio relative to a distance between the plurality of ultraviolet lamps and the cross-flow fan.

Thus, by adjusting the separation distance between the ultraviolet lamps and the distance between the ultraviolet lamp and the cross-flow fan, an air conditioner including a sterilization module that can uniformly irradiate ultraviolet light to a cross-flow fan having a long length in the direction of a rotation axis may be provided.

The sterilization module of the air conditioner according to the embodiment of the present disclosure may be configured such that a plurality of sterilization modules are arranged in a longitudinal direction of the cross-flow fan so as to correspond to a length of the cross-flow fan.

Thus, as the plurality of modalized sterilization modules are disposed to correspond to the cross-flow fan that varies in length depending on the capacity of the air conditioner, it possible to provide an air conditioner with scalability.

Details of other embodiments are included in the detailed description and the accompanying drawings.

### [Advantageous Effects]

According to the embodiments of the present disclosure, it is possible to provide an air conditioner including a sterilization module capable of irradiating ultraviolet light to a cross-flow fan of the air conditioner without any missing part while covering an entire length of the cross-flow fan.

According to the embodiments of the present disclosure, as a sterilization module is disposed at a lower portion of an upper case to facilitate a user's access upon removing a lower case exposed to an indoor space, it is possible to provide an air conditioner including a sterilization module with improved installation and access convenience.

According to the embodiments of the present disclosure, as a placement space of a sterilization module is produced through a simple bending or recess process regardless of the shape of typical air conditioner that has been manufactured and/or distributed, it is possible to provide an air conditioner including a sterilization module that is easily applicable to air conditioners that have been manufactured and/or distributed.

According to the embodiments of the present disclosure, as a sterilization module is disposed adjacent to a fan that causes a flow of air so as to sterilize the fan and is embedded so as not to interfere with the flow of air, it is possible to provide an air conditioner including a sterilization module that is disposed adjacent to a fan that causes a flow of air without affecting the flow of air.

According to the embodiments of the present disclosure, as a PCB bar is coupled to a lamp case to couple the lamp case to a module case, it is possible to provide an air conditioner including a sterilization module that is easy to manufacture.

According to the embodiments of the present disclosure, as the shape of an ultraviolet light outlet of a sterilization module is formed in consideration of the irradiation angle of ultraviolet light, it is possible to provide an air conditioner including a sterilization module capable of expanding an irradiation angle of ultraviolet light irradiated to a cross-flow fan in a limited inner space of the air conditioner while preventing the ultraviolet light, which can be harmful to a human body, from leaking to an outside of the air conditioner.

According to the embodiments of the present disclosure, by adjusting a separation distance between ultraviolet lamps and a distance between the ultraviolet lamp and a cross-flow fan, it is possible to provide an air conditioner including a sterilization module capable of uniformly irradiating ultraviolet light to a cross-flow fan having a long length in the direction of a rotation axis.

According to the embodiments of the present disclosure, by placing a plurality of modalized sterilization modules to correspond to a cross-flow fan that varies in length depending on the capacity of the air conditioner, it possible to provide an air conditioner with scalability.

The effects of the present disclosure are not limited to the effects described above, and other effects not mentioned will be clearly understood by those skilled in the art from the claims.

### [Brief Description of Drawings]

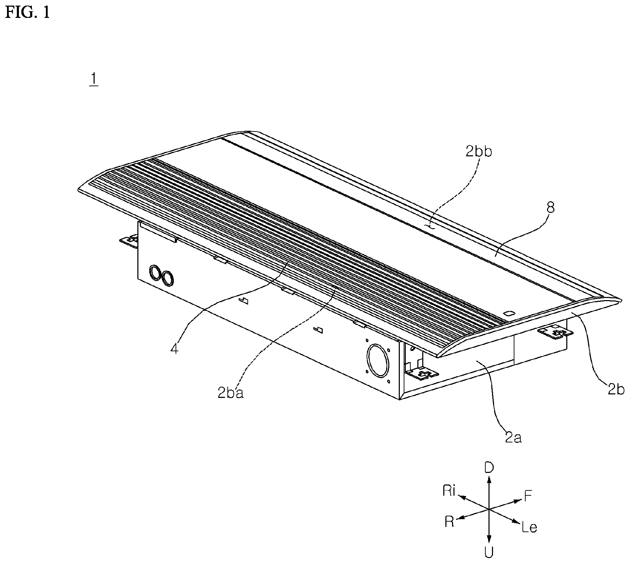

FIG. 1 is a perspective view of an air conditioner according to an embodiment of the present disclosure.

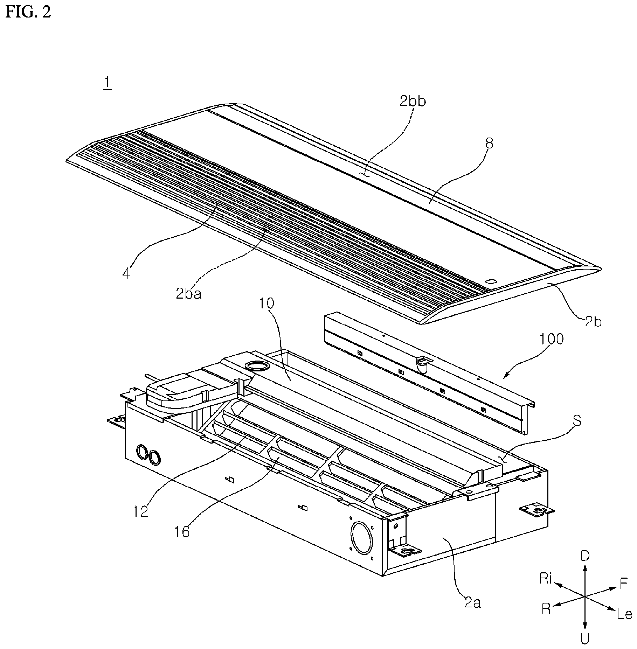

FIG. 2 is an exploded perspective view of an air conditioner according to an embodiment of the present disclosure.

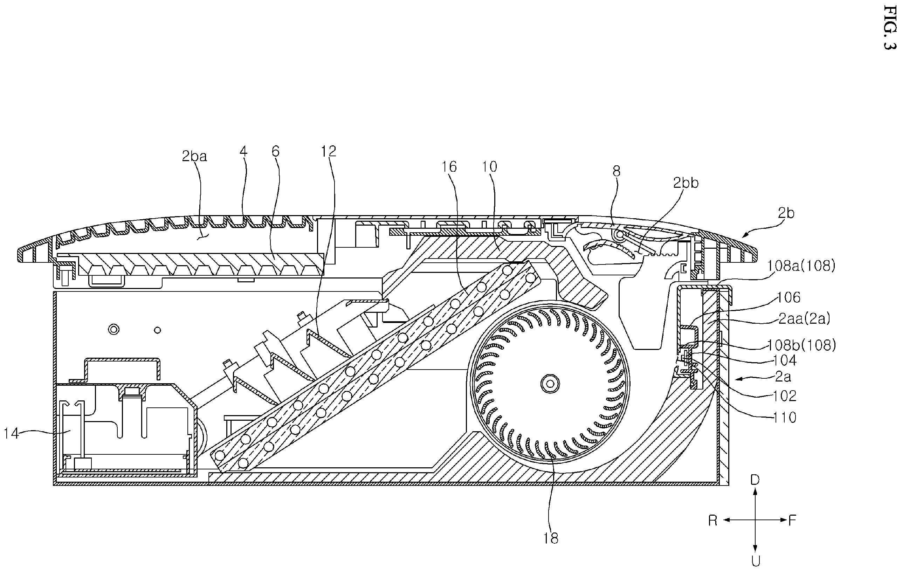

FIG. 3 is a side cross-sectional view of an air conditioner according to an embodiment of the present disclosure.

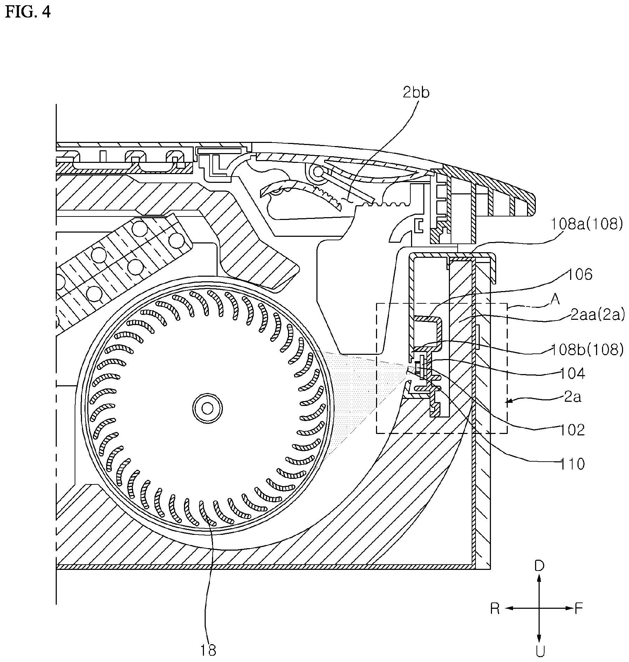

FIG. 4 is a side cross-sectional view of an air conditioner showing an irradiation angle of a sterilization module according to an embodiment of the present disclosure.
FIG. 5 is an enlarged view of a portion A of FIG. 4.

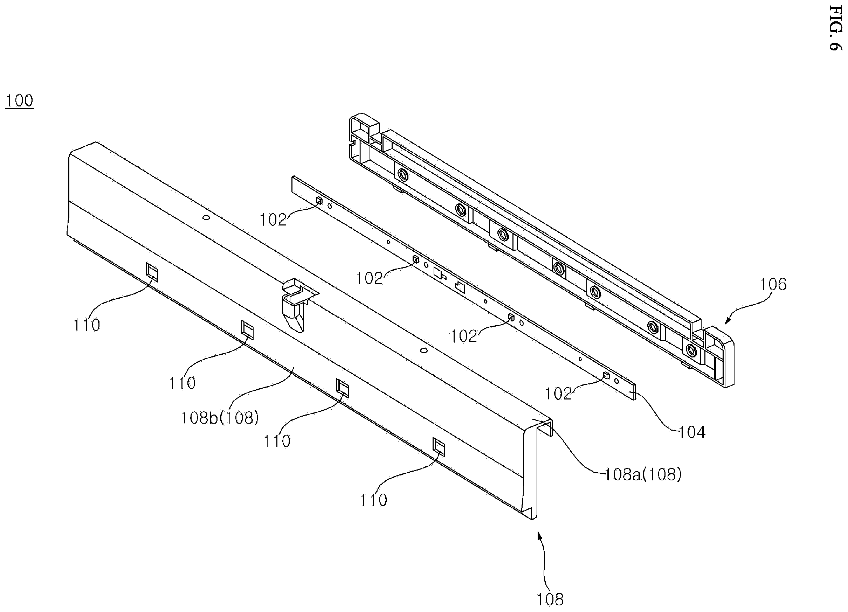

FIG. 6 is an exploded view of a sterilization module according to an embodiment of the present disclosure.

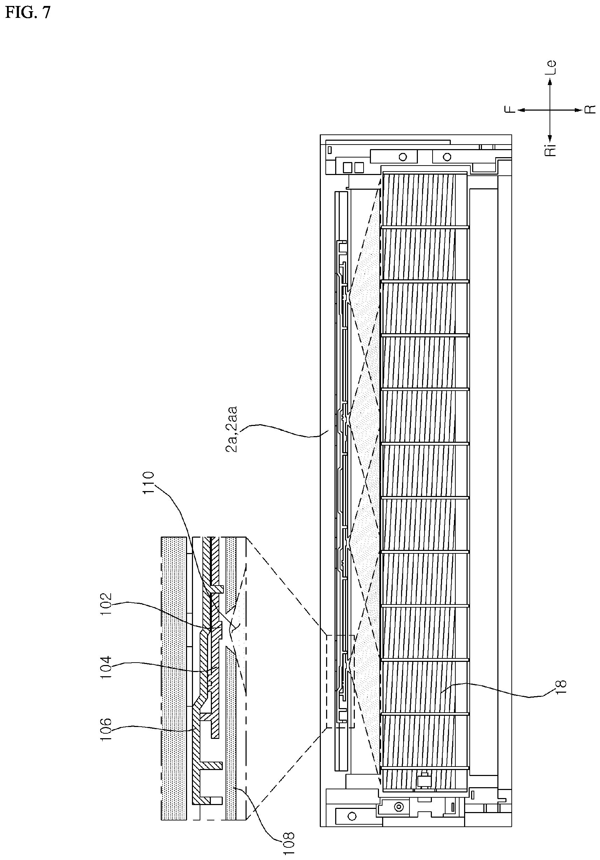

FIG. 7 is a planar view of an air conditioner showing an irradiation angle of a sterilization module according to an embodiment of the present disclosure.
FIG. 8 is a front view illustrating an ultraviolet lamp and a PCB bar, according to an embodiment of the present disclosure.
FIG. 9 is an enlarged planar view illustrating an ultraviolet lamp and a cross-flow fan, according to an embodiment of the present disclosure.
FIG. 10 is an exploded perspective view of an air conditioner including a plurality of sterilization modules, according to an embodiment of the present disclosure.
FIG. 11 is a schematic diagram illustrating the placement of a sterilization module according to an embodiment of the present disclosure.

### [Mode for the Invention]

In the drawings, in order to clearly and briefly describe the present disclosure, parts that are not related to the description will be omitted, and the same reference numerals are used for identical or extremely similar components throughout the specification.

It will be understood that the terms "comprises or includes", "has", etc. specify the presence of stated features, integers, steps, operations, elements, components, or combinations thereof, but do not preclude the presence or addition of one or more other features, integers, steps, operations, elements, components, or combinations thereof.

For example, it is obvious that '2' includes '2a' and/or '2b'.

Hereinafter, an air conditioner 1 according to embodiments of the present disclosure will be described with reference to the accompanying drawings.

A case 2 has an inlet 2ba and an outlet 2bb. The case 2 may define an inner space of the air conditioner 1. The case 2 may include an upper case 2a and a lower case 2b.

The upper case 2a may be mounted in the ceiling and may accommodate a cross-flow fan 18 therein. The upper case 2a may further accommodate therein a control box 14, a heat exchanger 16, and/or an auxiliary water collecting part 12.

The upper case 2a may include a guide housing 2aa that forms an air flow path from the cross-flow fan 18 to the outlet 2bb. The guide housing 2aa may be formed by protruding from the upper case 2a toward the inner space. The guide housing 2aa may be integrally formed with the upper case 2a. Air discharged from the cross-flow fan 18 may flow to the outlet 2bb along the surface of the guide housing 2aa.

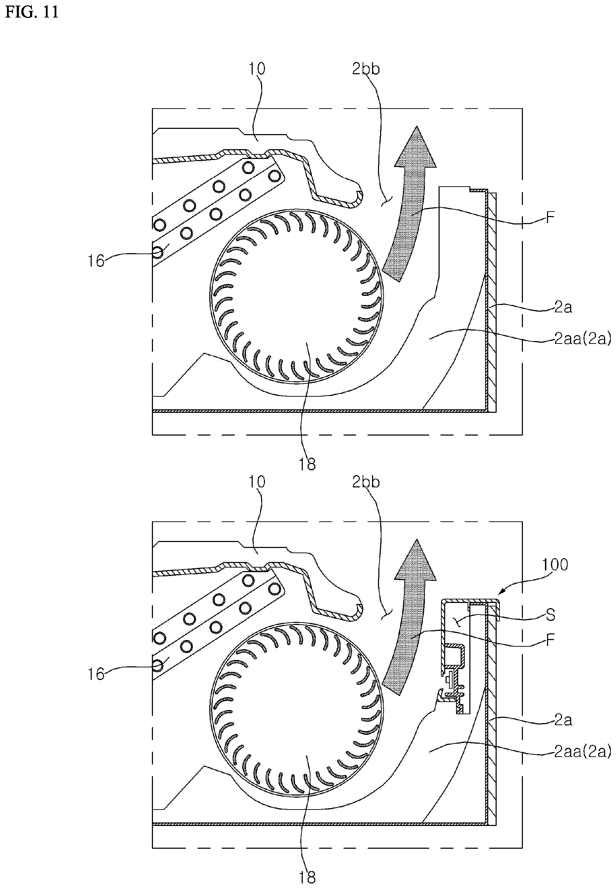

The guide housing 2aa may include a placement space S formed by one surface (or side) opposite the cross-flow fan 18 being bent (or recessed). The placement space S may have a shape corresponding to that of a sterilization module 100 to allow the sterilization module 100 to be embedded therein. The placement space S may be formed in such a manner that one surface adjacent to an air flow path F from the cross-flow fan 18 to the outlet 2bb is bent (or recessed) (see FIG. 11), so as not to interfere with the flow of air discharged from the cross-flow fan 18.

As the placement space S is formed by simply bending one surface of the guide housing 2aa, it may be manufactured through a simple bending process regardless of the shape of the typical air conditioner 1 that has been manufactured and/or distributed. Therefore, the sterilization module 100, which will be described later, is universally applicable to typical air conditioners 1 that have been manufactured and/or distributed.

The lower case 2b may be disposed under the upper case 2a so as to be exposed to an indoor space. The lower case 2b may be provided with the inlet 2ba and the outlet 2bb. As a portion of the lower case 2b is open, the inlet 2ba and the outlet 2bb may be formed at the indoor space side. The lower case 2b may accommodate therein a main water collecting part 10, a vane 8, a grille 4, and/or a filter 6.

The lower case 2b may be detachably coupled to the upper case 2a. Thus, by detaching the lower case 2b exposed to the indoor space, a user can easily access the upper case 2a or the inner space of the upper case 2a.

The cross-flow fan (CFF) 18 is disposed in the case 2 to cause a flow of air. As the cross-flow fan 18 operates, air in the indoor space is sucked into the air conditioner 1 through the inlet 2ba, and the sucked air is discharged to the outlet 2bb through the heat exchanger 16. The cross-flow fan 18 may have a predetermined length in the direction of a rotation axis.

The heat exchanger 16 is disposed in the case 2 to exchange heat with the flowing air. The heat exchanger 16 may be disposed to be inclined at a predetermined angle, so that condensed water on the surface, which is formed due to heat exchange, runs off.

The control box 14 may be disposed in the case 2, and may be provided therein with a control unit (PCB) (not shown) and a power source (not shown).

The main water collecting part 10 may be disposed at a lower end of the heat exchanger 16 so as to collect condensed water formed on the heat exchanger 16. The main water collecting part 10 may be a bowl-shaped structure to allow condensed water to be collected therein. A fan (not shown) may be disposed in the main water collecting part 10 to take in and drain condensed water.

The auxiliary water collecting part 12 may be disposed under the heat exchanger 16 along a longitudinal direction of the heat exchanger 16, so as to collect condensed water formed on the heat exchanger 16 and allow the collected condensed water to flow down to the main water collecting part 10.

The grille 4 may be disposed at the inlet 2ba to filter out foreign substances contained in the introduced air.

The filter 6 may be disposed on a downstream side of the grille 4 of the inlet 2ba, so as to remove foreign substances from the air that has passed through the grille 4.

The vane 8 may be disposed at the outlet 2bb to guide a direction of the discharged air.

The sterilization module 100 is disposed in the case 2 in the direction of the rotation axis of the cross-flow fan 18, and is configured to irradiate ultraviolet light to the cross-flow fan 18. That is, the sterilization module 100 is disposed such that a longitudinal direction of the sterilization module 100 is parallel to the direction of the rotation axis of the cross-flow fan 18. The cross-flow fan 18 may be sterilized by the ultraviolet light irradiated by the sterilization module 100. The sterilization module 100 includes a plurality of ultraviolet lamps 102 arranged to be spaced apart from each other in the direction of the rotation axis of the cross-flow fan 18. The ultraviolet lamp 102 may be, for example, a UV light source consisting of LED elements.

Accordingly, the sterilization module 100 may irradiate ultraviolet light to the cross-flow fan 18 while covering a length in the direction of the rotation axis of the cross-flow fan 18, thereby allowing the entire area of the cross-flow fan 18 to be thoroughly sterilized.

The sterilization module 100 may be disposed at a lower portion of the upper case 2a. The sterilization module 100 may be detachably coupled to the upper case 2a. For example, the sterilization module 100 may further include a hooking portion 108a that is engaged with the upper case 2a so as to couple the sterilization module 100 to the upper case 2a. The hooking portion 108a of the sterilization module 100 may be formed of an elastic member corresponding to the shape of the lower portion of the upper case 2a so as to be hooked to the lower portion of the upper case 2a. The hooking portion 108a of the sterilization module 100 may be a portion of a module case 108 described later.

As the sterilization module 100 is disposed at the lower portion of the upper case 2a to facilitate a user's access upon removing the lower case exposed to the indoor space, it is possible to improve the installation and access convenience of the sterilization module 100.

The sterilization module 100 may further include an embedded portion 108b embedded in the placement space S so as to couple the sterilization module 100. The embedded portion 108b of the sterilization module 100 may be embedded in the placement space S, which is formed by the guide housing 2aa being bent, so as not to interfere with the flow of air discharged from the cross-flow fan 18. The embedded portion 108b may be embedded in the placement space S to thereby couple the sterilization module 100 to the guide housing 2aa. The embedded portion 108b may be a portion of the module case 108 (see FIG. 11), which will be described later.

Thus, although the sterilization module 100 is disposed adjacent to the fan in order to sterilize the fan that causes a flow of air, as the sterilization module 100 is embedded so as not to interfere with the flow of air, it is possible to prevent a decrease in performance of the air conditioner 1.

The sterilization module 100 may further include a PCB bar 104 on which the plurality of ultraviolet lamps 102 are disposed and electrically connected to the plurality of ultraviolet lamps 102. The PCB bar 104, which is in the form of a commonly used bar, allows the plurality of ultraviolet lamps 102 (e.g., LED elements) to be arranged in a row and spaced apart from each other in the direction of the rotation axis of the cross-flow fan 18.

The sterilization module 100 may further include a lamp case 106 to which the PCB bar 104 is coupled. The lamp case 106 may be formed in the shape of a bar having a larger area than the PCB bar 104, so as to allow the PCB bar 104 to be accommodated therein. The lamp case 106 and the PCB bar 104 may share a plurality of bolt holes be bolted together. The lamp case 106 may protect the PCB bar 104 from external impact and foreign substances while firmly supporting the PCB bar 104.

The sterilization module 100 may further include the module case 108 to which the lamp case 106 is coupled. The module case 108 may be divided into the embedded portion 108b and the hooking portion 108a described above. The embedded portion 108b of the module case 108 may define a semi-closed space with one surface (or side) open to accommodate the lamp case 106, so that the lamp case 106 may be inserted through the open surface. The embedded portion 108b of the module case 108 may be embedded in the guide housing 2aa while being in contact with the one surface of the guide housing 2aa that defines the placement space S. The hooking portion 108a of the module case 108 may be a hook that extends from the lower side of the embedded portion 108b so as to be hooked to the upper case 2a. The module case 108 may protect the lamp case 106 from external impact and foreign substances while firmly supporting the lamp case 106. The module case 108 may be coupled to the upper case 2a to thereby couple the sterilization module 100 with the upper case 2a.

Thus, as the sterilization module 100 is easily assembled by coupling the PCB bar 104 to the lamp case 106 and then coupling the lamp case 106 to the module case 108, it is possible to improve manufacturability.

A plurality of opening holes 110 corresponding to the plurality of ultraviolet lamps 102 may be formed in the module case 108. Specifically, as the embedded portion 108b of the module case 108 accommodates the lamp case 106 to which the PCB bar 104 is coupled, the plurality of opening holes 110 may be formed at positions corresponding to the plurality of ultraviolet lamps 102 arranged on the PCB bar 104. Ultraviolet light emitted by the plurality of ultraviolet lamps 102 through the plurality of openings 110 may be irradiated to the cross-flow fan 18.

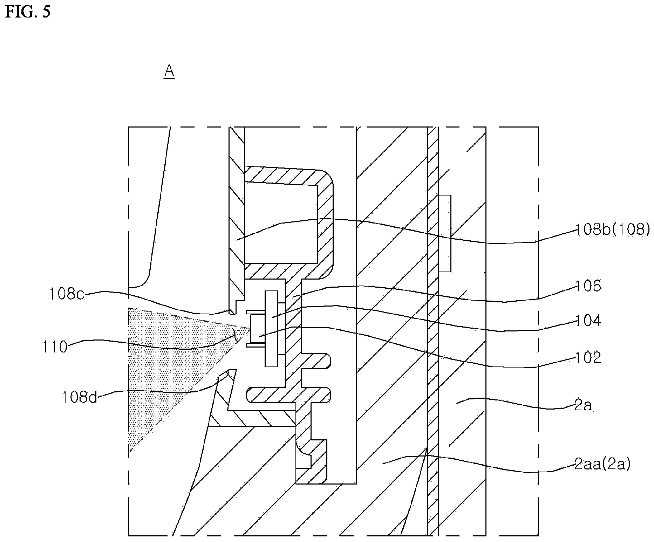

The module case 108 may include a protruding portion 108c (see FIG. 5) that protrudes from the side of the inlet 2ba and the outlet 2bb to the opposite side of the inlet 2ba and the outlet 2bb in the plurality of opening holes 110 so as to limit an irradiation angle of the side of the inlet 2ba and the outlet 2bb. The protruding portion 108c may prevent ultraviolet light of the sterilization module 100 discharged through the opening hole 110 from leaking toward the side of the inlet 2ba and the outlet 2bb.

The module case 108 may include an inclined portion 108d (see FIG. 5) formed at the opposite side of the inlet 2ba and the outlet 2bb in the plurality of opening holes 110 so as to expand an irradiation angle of the opposite side of the inlet 2ba and the outlet 2bb. The inclined portion 108d may widen the irradiation angle so that the ultraviolet light of the sterilization module 100 discharged through the opening hole 110 is irradiated to a wider area of the cross-flow fan 18.

Thus, by forming the shape of an ultraviolet light outlet of the sterilization module 100 in consideration of the irradiation angle of ultraviolet light, it is possible to expand the irradiation angle of ultraviolet light irradiated to the cross-flow fan 18 in the limited inner space of the air conditioner 1 while preventing ultraviolet light, which can be harmful to a human body, from leaking to the outside of the air conditioner 1.

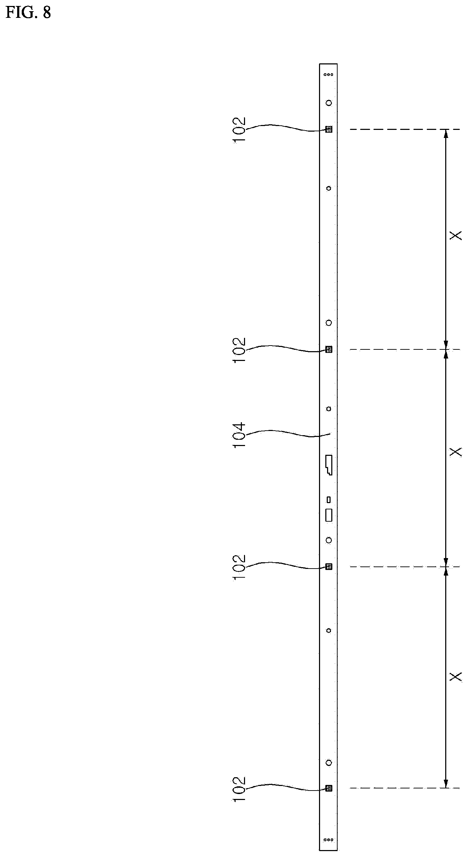

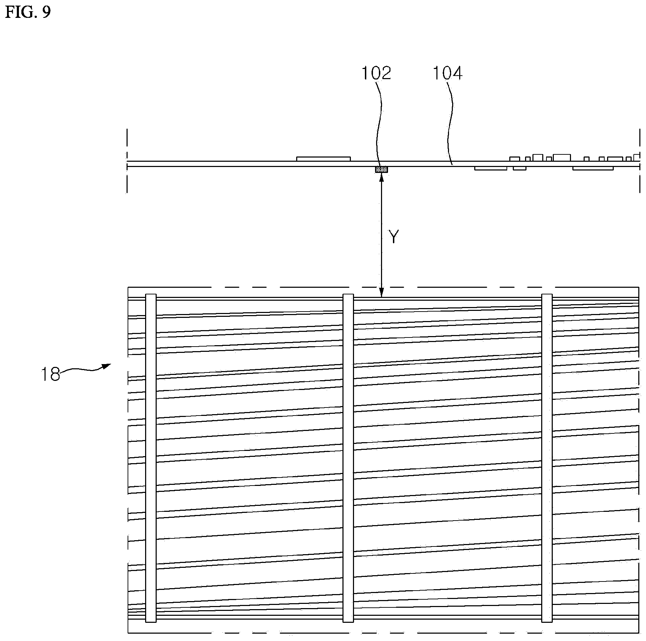

A separation distance X between adjacent ultraviolet lamps 102 of the plurality of ultraviolet lamps 102 may have a predetermined ratio relative to a distance Y between the plurality of ultraviolet lamps 102 and the cross-flow fan 18. The predetermined ratio may be an experimental value determined in consideration of the uniformity of ultraviolet light irradiated to the cross-flow fan 18. For example, if X is 110 to 140 mm, Y may be 40 mm (see FIGS. 8 and 9).

Thus, by adjusting the separation distance between the ultraviolet lamps 102 and the distance between the ultraviolet lamp 102 and the cross-flow fan 18, it is possible to evenly irradiate ultraviolet light to the cross-flow fan 18 having a long length in the direction of the rotation axis to thereby improve the performance of the sterilization module 100.

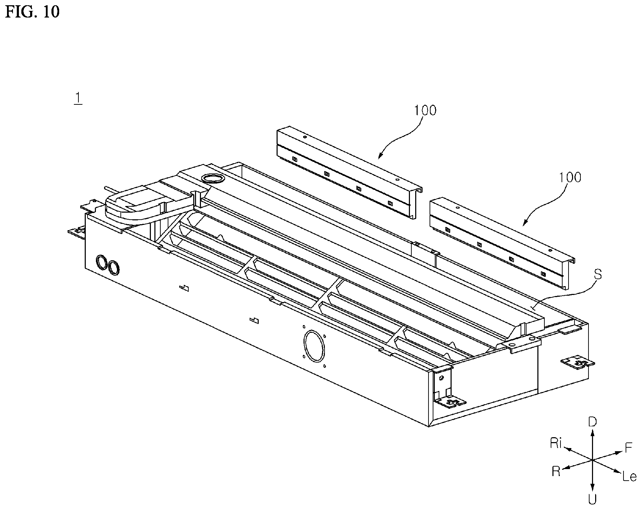

A plurality of sterilization modules 100 may be arranged in the longitudinal direction of the cross-flow fan 18 so as to correspond to the length of the cross-flow fan 18 (i.e., the length in the direction of the rotation axis of the cross-flow fan 18) (see FIG. 10).

For example, the length of the cross-flow fan 18 increases as the capacity or size of the air conditioner 1 increases, however, it can be easily addressed by additionally placing the modularized sterilization module 100 in the longitudinal direction of the cross-flow fan 18.

Thus, as the plurality of modularized sterilization modules 100 are disposed to correspond to the cross-flow fan 18, which increases in length depending on the capacity of the air conditioner 1, it is possible to achieve scalability of the sterilization module 100.

The accompanying drawings are used to help easily understand various embodiments of the present disclosure and it should be understood that the embodiments presented herein are not limited by the accompanying drawings, and the present disclosure should be construed to extend to any alterations, equivalents and substitutes in addition to those which are particularly set out in the accompanying drawings.

Similarly, while operations are depicted in the drawings in a particular order, this should not be understood as requiring that such operations be performed in the particular order shown or in sequential order, or that all illustrated operations be performed, to achieve desirable results. In certain circumstances, multitasking and parallel processing may be advantageous.

While the present disclosure has been particularly shown and described with reference to exemplary embodiments thereof, it is clearly understood that the same is by way of illustration and example only and is not to be taken in conjunction with the present disclosure. It will be understood by those skilled in the art that various changes in form and details may be made therein without departing from the subject matter and scope of the present disclosure.

For example, although the present disclosure has been described that the placement space S is formed on the side of the air flow path from the cross-flow fan 18 to the outlet 2bb so that the sterilization module 100 is embedded in the placement space S, the technical idea of the present disclosure is not limited thereto. For instance, the sterilization module 100 may be embedded on the side of the air flow path from the inlet 2ba to the cross-flow fan 18.

For example, the present disclosure has been described by using a ceiling mounted air conditioner 1 in which the cross-flow fan 18 is generally used, but the technical idea of the present disclosure is not limited thereto. For instance, the sterilization module 100 according to the embodiments of the present disclosure is applicable to any type of air conditioner 1, regardless of the type of air conditioner 1, so long as the cross-flow fan 18 is employed therein.

## Claims

1. An air conditioner comprising:
a case that defines an inlet and an outlet;
a cross-flow fan that is disposed in the case and causes a flow of air;
a heat exchanger that is disposed in the case and configured to exchange heat with flowing air; and
a sterilization module that is disposed in the case in a direction of a rotation axis of the cross-flow fan, the sterilization module being configured to irradiate ultraviolet light to the cross-flow fan,
wherein the sterilization module comprises a plurality of ultraviolet lamps arranged to be spaced apart in the direction of the rotation axis of the cross-flow fan.

2. The air conditioner of claim 1, wherein the case comprises:
an upper case accommodated in a ceiling and in which the cross-flow fan is disposed; and
a lower case disposed under the upper case so as to be exposed to an indoor space and in which the inlet and the outlet are disposed, and
wherein the sterilization module is disposed at a lower portion of the upper case.

3. The air conditioner of claim 2, wherein the lower case and the sterilization module are detachably coupled to the upper case.

4. The air conditioner of claim 3, wherein the sterilization module further comprises a hooking portion that is hooked to the upper case so as to couple the sterilization module to the upper case.

5. The air conditioner of claim 4, wherein the upper case comprises a guide housing that forms an air flow path from the cross-flow fan to the outlet,
wherein the guide housing includes a placement space formed as one surface opposite the cross-flow fan being bent, and
wherein the sterilization module further comprises an embedded portion embedded in the placement space to couple the sterilization module.

6. The air conditioner of claim 1, wherein the sterilization module further comprises a PCB bar on which the plurality of ultraviolet lamps are disposed, the PCB bar being electrically connected to the plurality of ultraviolet lamps.

7. The air conditioner of claim 6, wherein the sterilization module further comprises:
a lamp case to which the PCB bar is coupled; and
a module case to which the lamp case is coupled, the module case having a plurality of opening holes that correspond to the plurality of ultraviolet lamps.

8. The air conditioner of claim 7, wherein the module case comprises:
a protruding portion protruding from a side of the inlet and the outlet in the plurality of opening holes so as to limit an irradiation angle of the side of the inlet and the outlet; and
an inclined portion formed on an opposite side of the inlet and the outlet in the plurality of opening holes so as to expand an irradiation angle of the opposite side of the inlet and the outlet.

9. The air conditioner of claim 1, wherein a separation distance between adjacent ultraviolet lamps of the plurality of ultraviolet lamps has a predetermined ratio relative to a distance between the plurality of ultraviolet lamps and the cross-flow fan.

10. The air conditioner of claim 1, wherein the sterilization module is configured such that a plurality of sterilization modules are arranged in a longitudinal direction of the cross-flow fan so as to correspond to a length of the cross-flow fan.
